# EUROPEAN PATENT APPLICATION

(11) **EP 1 254 953 A1**
(43) Date of publication of application: **06.11.2002**
(21) Application number: 00987772.1
(22) Date of filing: 28.12.2000
(51) Int. Cl.: C12N 5/08, A61K 35/12, A61K 39/39, A61K 35/74, A61P 37/04, A61P 35/00, A61K 39/00, A61P 43/00

(54) **MATURATION-PROMOTING AGENT FOR IMMATURE DENDRITIC CELLS**

(30) Priority: 28.12.1999 JP 37395099
(71) Applicant: Toyoshima, Kumao, Osaka 534-0027 (JP); Seya, Tsukasa, Nara-shi, Nara 631-0022 (JP); Hayashi, Akira, Suita-shi, Osaka 565-0862 (JP); Azuma, Ichiro, Sapporo-shi Hokkaido 005-0012 (JP)
(72) Inventor: SEYA, Tsukasa, Nara-shi, Nara 631-0022 (JP); HAYASHI, Akira, Suita-shi, Osaka 565-0862 (JP); AZUMA, Ichiro, Sapporo-shi, Hokkaido 005-0012 (JP); TSUJI, Shoutaro, Higashiosaka-shi, Osaka 577-0066 (JP); MATSUMOTO, Misako, Ikoma-shi, Nara 630-0121 (JP)
(74) Representative: VOSSIUS & PARTNER
(86) International application number: JP0009358
(87) International publication number: WO01048154

(57) **Abstract**

This invention relates to dendritic cells that are matured by utilizing a cell wall skeleton of a Gram positive bacteria-CWS and a process for maturing said cells, as well as a composition for accelerating the maturation of immature dendritic cells comprising a Gram positive bacteria-CWS as an essential component.

## Description

### FILED OF THE INVENTION

This invention relates to dendritic cells that are matured by utilizing a cell wall skeleton of Calmette-Guerin strain of *Mycobacterium bovis* (referred to as BCG-CWS hereinafter) and a process for maturing said cells, as well as a composition for accelerating the maturation of immature dendritic cells comprising BCG-CWS as an essential component.

### BACKGROUND ART

The numbers depicted in the brackets herein refer to those of references collectively described at the end of the present specification.

Immune system has developed with continuous exposure to foreign materials such as bacteria, virus and fungi. In humans, these materials frequently function as activators of host innate immunity, and allow progenitor cells to mature into professional antigen-presenting cells (APC) including macrophage and dendritic cells (DC) (1, 2) so that the mature macrophage/dendritic cells can present ingested antigens to T cells. Such cellular responses induced by foreign materials are known as innate immune system. Various evidences have suggested that innate immune system plays a instructive role in the activation of lymphocytes, which system is known as acquired immune system (1-3).

Bacillus Calmette-Guerin strains of *Mycobacterium bovis* (bovine tubercle bacilli) are tuberculosis vaccine strains, and are almost non-pathogenic although they retain immunogenicity to tubercle bacilli (4). Several reports have suggested that phagocytosis of live BCG/mycobacterial cells is a potent inducer of maturation of dendritic cells (5-7). Human and murine immature dendritic cells exhibit a potent antigen-presenting activity through the uptake of live BCG bacteria (5, 6). Such a potent antigen-presenting activity of dendritic cells could be provided due to the uptake of soluble antigens other than BCG. These studies have led to a consensus that live BCG bacteria serve as an immune potentiator of lymphocytes via the maturation of dendritic cells, although the studies were conducted in different experimental conditions. In fact, human dendritic cells infected with live BCG or *M. Tuberculosis* bacteria (human tubercle bacilli) resulted in homotypic aggregation, facilitating the secretion of inflammatory cytokines including TNF-α, IL-1β and IL-12, and the up-regulation of CD40, CD80 and MHC class I molecules (6, 7).

The recent studies as shown above may interpret an observations previously obtained. BCG has been used as an adjuvant effective for the active immunotherapy of various cancers (8, 9). In humans, immunotherapy with live BCG bacteria has been employed in the treatment and prophylaxis of transitional cell carcinomas of the bladder and the urinary tract for 20 years and more, and has demonstrated a good prognosis (10). Live BCG bacteria were also effective as a vaccination adjuvant for immunization of irradiated colon cancer cells (11, 12). It has been reported that injection of BCG into the tumors transplanted into mouse and guinea-pig was effective in tumor regression (13-16). However, no explicit hypothesis has been proposed regarding any component of BCG responsible for anti-tumor immunity, or any mechanism by which BCG can potentiate the host immune system.

BCG-CWS has been found to be an adjuvant effective for antibody production in animal studies (17-19). Typical delayed type hypersensitivity (DTH) could be also produced by intracutaneous injection of BCG-CWS as shown with live BCG bacteria. Immunotherapy with BCG-CWS has demonstrated a good prognosis without any symptomatic infection in many cancer patients (20, 21). Major constituents of BCG-CWS are arabinogalactan, mycolic acid, and peptidoglycan (17). Taken together, any one of these molecules or a combination thereof should play an important role in adjuvant function.

Human dendritic cells culturing protocols that were recently established (22-25) makes it possible to conduct *in vitro* analysis of the effect of immunomodulators on the function of dendritic cells. The inventors of the present application continued such analysis using BCG-CWS and the human cultured dendritic cells, so as to elucidate the function of a Gram positive bacteria-CWS on the dendritic cells culture system. As a result, the inventors found that a non-infectious agent, Gram positive bacteria-CWS, could accelerate the maturation of immature dendritic cells. It has been known that IL-1β, TNF-α, and lipopolysaccharide mature immature dendritic cells. Dendritic cells matured with a Gram positive bacteria-CWS are characterized in that they substantially maintain a phagocytic ability.

Based on the findings as shown above, the present invention has been accomplished.

### DISCLOSURE OF THE INVENTION

The subjects of the present invention are described as follows.
(1) A matured dendritic cell that maintains a phagocytic ability.
(2) A process for maturing an immature dendritic cell which comprises employing a Gram positive bacteria-CWS, said process providing a matured dendritic cell that maintains a phagocytic ability.
(3) A process for inducing the expression of TNF-α, CD40, CD71, CD83, CD80, and/or CD86 in dendritic cells, which comprises employing a Gram positive bacteria-CWS.
(4) A composition for accelerating the maturation of immature dendritic cells, which comprises a Gram positive bacteria-CWS as an active ingredient.
(5) A composition for accelerating the induction of TNF-α, IL-12p40, and/or IL-6, which comprises a Gram positive bacteria-CWS as an active ingredient.
(6) A composition for accelerating the expression of CD40, CD71, CD83, CD80, and CD86, which comprises a Gram positive bacteria-CWS as an active ingredient.
(7) An immune adjuvant which comprises a matured dendritic cell that maintains a phagocytic ability.
(8) A composition for immunotherapy of a cancer, which comprises a matured dendritic cell that maintains a phagocytic ability, said composition being used together with an anti-cancer vaccine.

The term "matured dendritic cell" as used herein refers to dendritic cells that have an antigen-presenting ability and a costimulating activity so as to be capable of activating antigen-specific T cells. Specifically, the maturation is determined by expression pattern of various costimulatory molecules (for example, presence or absence of CD83 expression). In general, T cells are activated when an antigen is presented on an antigen-presenting cell, and an activation signal from costimulatory molecules is received. As such, antigen-presenting cells such as macrophages and B cells do not activate T cells until costimulatory molecules are induced and expressed. The matured dendritic cells constitutively express costimulatory molecules at a high level, and are differentiated from other major antigen-presenting cells in this point.

Gram positive bacteria as used in the invention are exemplified *by Mycobacteriaceae, Nocardiaceae, Corynebacteriaceae,* and the like. Preferred examples include BCG of *Mycobacterium bovis.*

The followings are the description of the invention taking up BCG-CWS as an example.

### SUMMARY OF THE INVENTION

Infection with bacillus Calmette-Guerin (BCG) *Mycobacteria* strain induces the maturation of human immature dendritic cells (iDC) into matured dendritic cells with a potent antigen-presenting activity (mDC). The inventors identified the constituents of BCG responsible for the maturation of dendritic cells (DC). Specifically, it was found that the cell wall skeleton of BCG (BCG-CWS) comprising mycolic acid, arabinogalactan and peptidoglycan demonstrated a maturation activity on DC. BCG-CWS was bound to iDC, but not any lymphocytes, and was phagocytosed by iDC. Dendritic cells treated with BCG-CWS (referred to as DC^{BCG}) showed an elongated shape, high expression levels of CD80 and CD86, secretion of IL-12p40, and an extensive allogenic mixed lymphocyte reaction (MLR), all of which are the typical characteristics reported for the mDC. DC^{BCG}, however, are different from conventional mDC prepared with IL-1β, LPS or live BCG bacteria in terms of both biological and functional activities. Especially, DC^{BCG} maintains a phagocytic ability, and demonstrates a potent antigen-presenting activity at the same time. Although live BCG infection is currently considered to be an essential factor for the DC maturation, the present invention revealed that it is not required. The inventors have demonstrated that the uptake of one of three BCG-CWS constituents or a combination thereof was sufficient for the maturation into unique BCG-CWS-derived mDC. These findings, together with our previous findings that adjuvants containing either dead *Mycobacteria* or BCG-CWS serve as a potent innate immune activator and they are effective for tumor immunotherapy, suggest that the DC maturation, but not infection with BCG nor phagocytic uptake of BCG, could contribute to host immune activation by BCG.

### DETAILED DESCRIPTION OF THE INVENTION

The inventors demonstrated that cell wall skeleton of BCG, BCG-CWS, was able to differentiate human iDC into a more active form, which is herein referred to as DC^{BCG}. While having some of the properties of conventional mDC, DC^{BCG} also possess the unique characteristics including pseudopodia, up-regulation of the mDC marker CD83 and costimulators CD80 and CD86, secretion of IL-12p40, IL-6, and TNF-α, and induction of allogenic MLR. These results suggest that the purified cell wall constituents containing mycolic acid, arabinogalactan and peptidoglycan are sufficient to mature iDC into mDC.

Several reports describe the properties of human and murine DCs laden with live *Mycobacteria* (or BCG) (5-7). Live mycobacteria show more ability to mature iDC than dead mycobacteria. Live mycobacteria or BCG facilitate marked aggregation but not elongation of the infected DC. mDC that has been matured with live bacteria has a decreased or lost phagocytic ability (6, 7). Although different conditions were employed in these reports, it has been commonly understood that phagocytosis of live bacteria is a pivotal factor in inducing the DC maturation (6, 7). Contrarily, the present study of the inventors does not support this generally accepted view. The inventors have demonstrated that pure constituents of cell wall skeleton are sufficient to mature iDC into mDC. For the maturation of DC with BCG-CWS, phagocytic uptake of BCG-CWS was not critical in the maturation. Both iDC with or without BCG-CWS were matured with TNF-α secreted from the DC. The process was minimally dependent on direct intercellular events between matured and immature DCs, and thus cell-to-cell attachment was not essential for the DC maturation. TNF-α was an essential factor for the DC maturation even in the presence of BCG-CWS. A possible interpretation about these results is that BCG receptors responsible for phagocytosis are distinct from those for TNF-α secretion and DC maturation, and that the cell-wall constituents may provide ligands for both receptors. Mycobacteria contain a number of immunomodulators including TDM (trehalose dimycolate), LAM (lipoarabinomannan) and dDNA, in addition to the cell-wall components, mycolic acid, arabinogalactan and peptidoglycan. It should be noted that LAM is a potent immunosuppressor (32-34). TDM (35, 36) and dDNA (37, 38) also possess biological activities. Dead bacteria containing these factors could have reduced the maturing activity of BCG-CWS on DCs. Since the inventors almost completely eliminate TDM, LAM or dDNA from our preparation of BCG-CWS, the factors responsible for the maturation of iDC into mDC were most likely enriched in our BCG-CWS reparation.

In general, immune adjuvants including FCA (Freund's complete adjuvant) contain dead *M. tuberculosis.* FCA induces T cell-mediated immune responses and antibody-production more potently than FIA (Freund's incomplete adjuvant), which is a mineral oil without bacterial components and is traditionally used as a primary adjuvant (39). Immune activation or adjuvant potency by FCA has been mainly determined in animal experiments. The factors required for potent adjuvant activity have not been well defined yet. The results herein suggest that the DCs maturing activity of the BCG-CWS components may contribute in part to the potency of FCA as an adjuvant.

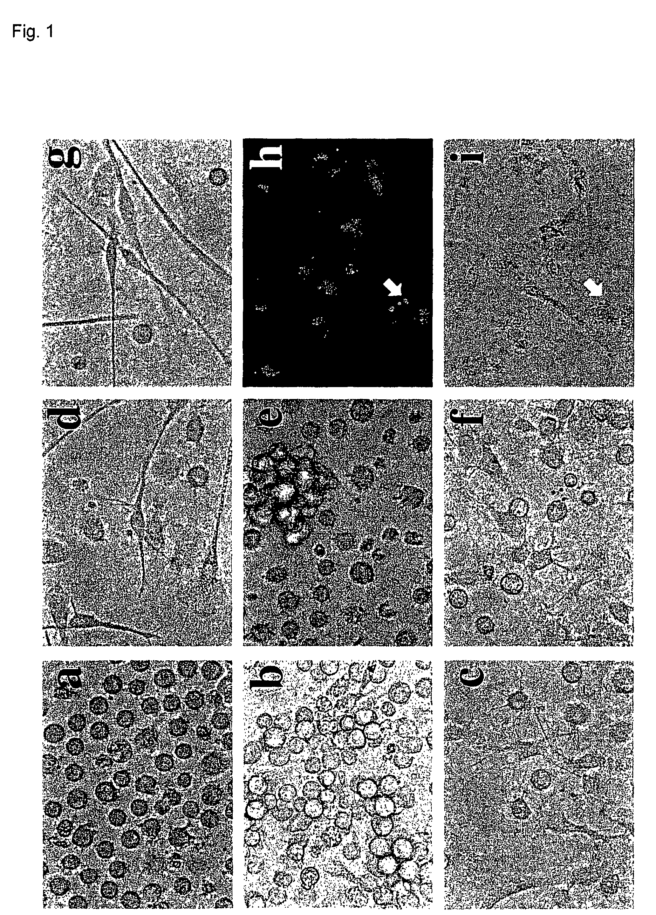

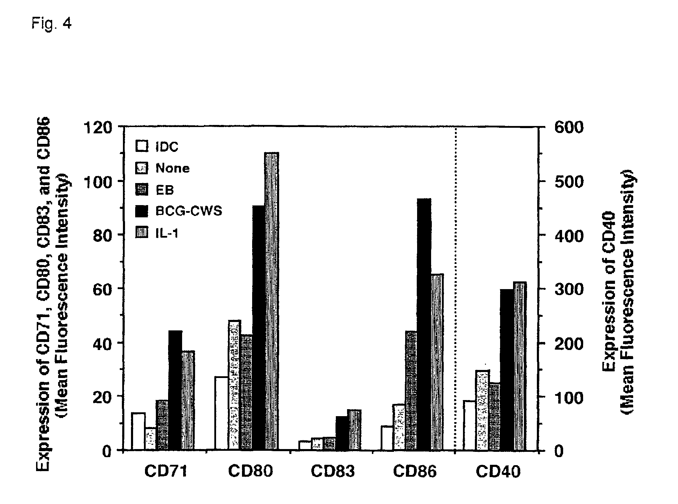

Foreign materials such as LPS, and host cell mediators including IL-1β, TNF-α and CD40L have been reported to act as DC maturators (29). Although these stimulators have been reported to be a typical maturation inducer for DC, mDC obtained by the treatment with these reagents did not have all of the morphological, flowcytometric and functional properties of DC^{BCG}. In contrast to BCG-CWS, LPS induced mDC with floating aggregation but not with extension (Fig. 1), and demonstrated a greater up-regulation of CD80, CD83 and CD86 during the phase of DC maturation. Again compared to BCG-CWS, IL-1β usually induced less elongation, and induced more expression of CD80 than CD86 (Figs. 1 and 4). DC^{BCG} resembled the TNF-α-derived matured DC most nearly, and TNF-α was found to be a necessary factor for maturation of iDC in BCG-CWS treatment. It should be noted that factors other than TNF-α may participate in DC maturation. Major differences between DC^{BCG} and TNF-α-treated DC include intracellular milieu, particularly phagolysosome formation. In DC^{BCG}, BCG-CWS was incorporated into phagosomes (Fig. 1h). The condition of phagosomes/lysosomes in these DC may be critical in antigen-processing and -transportation, and thereby affect antigen-presentation. In fact, DC^{BCG} retained phagocytic capacity whereas TNF-α-treated mDC did lose it.

An attractive approach for determining a mechanism by which BCG-CWS involves DC maturation, is to identify its receptor and search for a signaling pathway. The LPS signaling receptor was recently identified to be Toll-like receptor (TLR), TLR2 (40) and/or 4 (41, 42). TLR family proteins are currently believed to be receptors for materials of bacterial origin (3). TLR proteins share a similar cytoplasmic region with IL-1 receptor family proteins, and their stimulation commonly results in activation of NF_{K}B (3, 41). Recently, TLR2 has been reported to serve as a receptor for bacterial peptidoglycan (43). Indeed, BCG-CWS is a bacterial component containing a peptidoglycan. If TLR is a BCG-CWS receptor in DC maturation, most of DC maturation inducers should share the TLR/IL-1 receptor family or at least NF_{K}B activation in iDC. It will be important to test whether or not BCG-CWS-medicated cell activation is abolished in TLR- or Myd88-knockout mice, which fail to respond to LPS through TLR (44, 45).

It could be concluded that DC maturation profiles depend upon respective maturation reagent used. Examples of foreign materials, BCG-CWS and LPS, provided different results in DC maturation. Mediators of host origin also induced distinct DC maturation profiles. These findings lead to the prospect that even if activation of NF_{K}B is essential for DC maturation, there may be a number of complicated maturation courses and stages relative to iDC.

Although it has been known for a long time that BCG activates host immune system, little is known regarding molecular mechanism of BCG-mediated immune activation. Live BCG bacteria were found to induce DC maturation (5-7). Additionally, live BCG bacteria have been used for immunotherapy for bladder cancer in human (10). Immunotherapy with BCG-CWS has also demonstrated a good prognosis without any sign of infection in many cancer patients, which has not widely accepted (20, 21, 46). Our present study suggests that noninfectious BCG-CWS can be functionally substituted for live BCG bacteria at least as an inducer of iDC maturation. BCG-CWS can contribute to the induction of tumor immunity due to its ability to induce DC maturation although BCG-CWS itself is unlikely to be equivalent to live BCG bacteria in immunotherapy.

The present invention provides matured dendritic cells that maintain a phagocytic ability. Conventional matured dendritic cells do not have a phagocytic ability, and can not integrate an antigen. Contrary to those cells, dendritic cells that have been matured with a gram-positive cell-CWS can integrate and present an antigen. As a result, mere administration of an antigen to dendritic cells matured with a gram-positive cell-CWS provides the efficient induction of T cells that are reactive to the antigen.

### BRIEF DESCRIPTION OF DRAWINGS

Figure 1 is a photograph substitute for drawings showing morphology of dendritic cells and phagocytosis of FITC-BCG-CWS.

iDC cultured with GM-CSF and IL-4 for 6 days were incubated for 2 days (a-g) or 7 hours (h, i) in a medium containing GM-CSF (500 U/ml) and the following materials: (a) IL-4 (100 IU/ml), (b) GM-CSF alone (none), (c) IL-1β (100 ng/ml), (d) TNF-α (100 IU/ml), (e) LPS (10 ng/ml), (f) emulsion buffer (15 µl/ml), (g) BCG-CWS (15 µg/ml), (h) FITC-BCG-CWS (15 µg/ml), (i) FITC-BCG-CWS (15 µg/ml). Magnification: 400 x. The cells were observed under a microscope (a-g), a light fluorescence microscope (h), or a phase-shift microscope ((i), the same field as (h)). Arrows show FITC-labeled BCG-CWS binding to the surface of a floating cell.

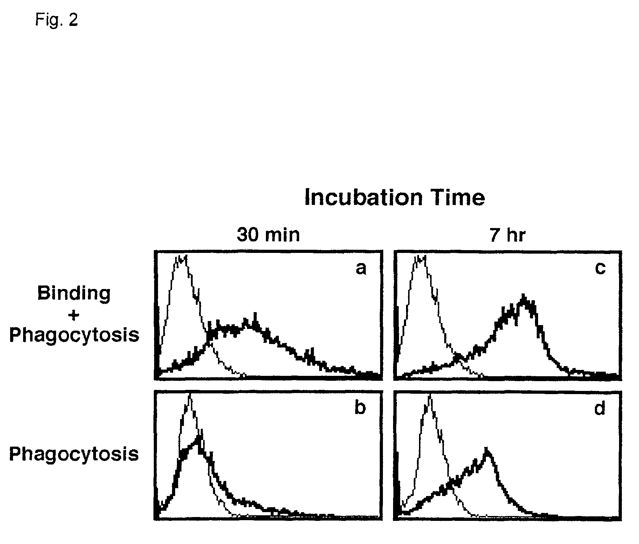

Figure 2 is a graph showing the result of flow cytometric analysis of FITC-BCG-CWS phagocytosis by iDC.

iDC cultured as in Figure 1 were incubated for 0.5 hours or 7 hours in a medium containing with GM-CSF (500 IU/ml) and FITC-BCG-CWS (15µg/ml). The cells were harvested by pipetting at 4 °C, and phagocytosed particles were analyzed by flowcytometry as described in the Methods section. Thin lines show self-fluorescence of cells and bold lines reflect fluorescence of FITC-BCG-CWS particles which are bound and/or phagocytosed. Total fluorescence intensities (panels a and c) and those quenched with trypan blue (panels b and d) are shown. Three experiments were performed and a representative one is shown.

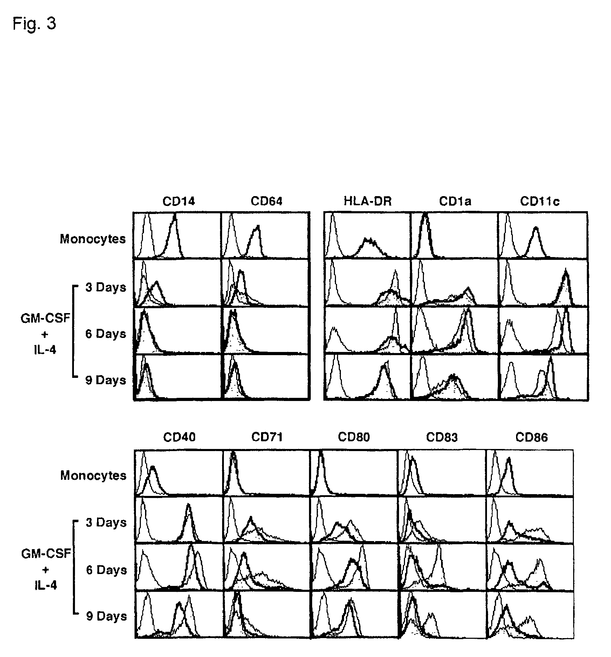

Figure 3 is a graph showing cell surface phenotypes of DC when exposed to BCG-CWS.

iDC were prepared by culture with GM-CSF and IL-4 for either 3, 6, or 9 days and were also incubated for an additional 2 days in medium containing with GM-CSF (500 IU/ml) and BCG-CWS (15 µg/ml). The cells were harvested and analyzed by flow cytometry as described in the Methods section. Broken lines in the histograms show nonspecific fluorescence by subclass control monoclonal antibody. Fluorescence for the indicated antigens on DC before and after BCG-CWS treatment are shown by bold lines and shaded areas, respectively. The experiment was performed three times with similar results.

Figure 4 is a graph showing levels of surface markers on iDC and those treated with reagents.

iDC prepared as in Figure 1 were incubated for 2 days in medium containing GM-CSF (500 IU/ml) and following materials: IL-4 (100 IU/ml), GM-CSF alone (none), emulsion buffer (15 µl/ml), BCG-CWS (15 µg/ml), IL-1β (100 ng/ml). Cells were harvested and analyzed by flow cytometry as described in the Methods section. The levels of CD40, CD71, CD80, CD83, and CD86 on DC were measured. Values are expressed as mean fluorescence intensity (MFI) measured by flow cytometer, and the mean values of subclass control monoclonal antibodies were negligible (MFI: ∼3-4). The experiment was performed three times and a representative experiment is shown.

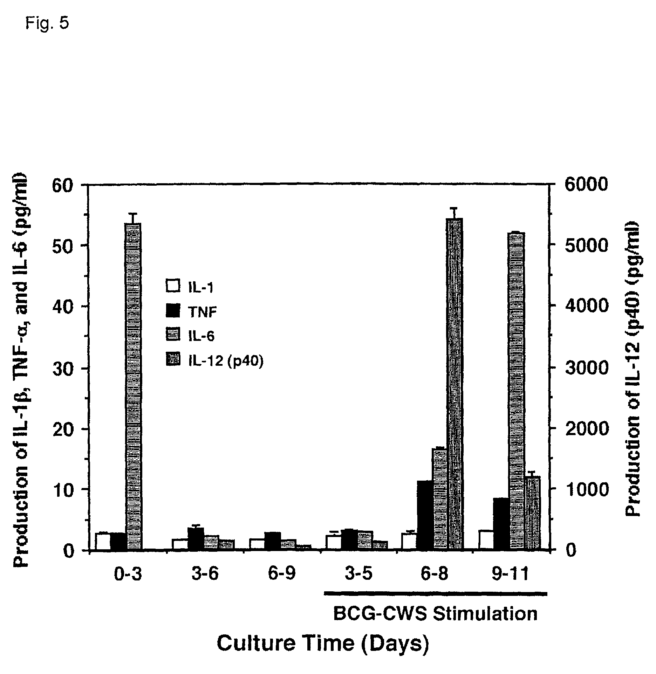

Figure 5 is a graph showing cytokine production by iDC and DC^{BCG}.

Monocytes were cultured in medium containing GM-CSF (500 IU/ml) and IL-4 (100 lU/ml), and the medium was collected every 3 days (0-3, 3-6, 6-9). iDC cultured for 3, 6, or 9 days were incubated in medium containing with GM-CSF (500 IU/ml) and BCG-CWS (15 µg/ml), and each medium was collected after 2 days respectively (3-5, 6-8, 9-11). The concentrations of each cytokine in the medium were determined by ELISA, and values represent the mean ± SD of triplicate determinations.

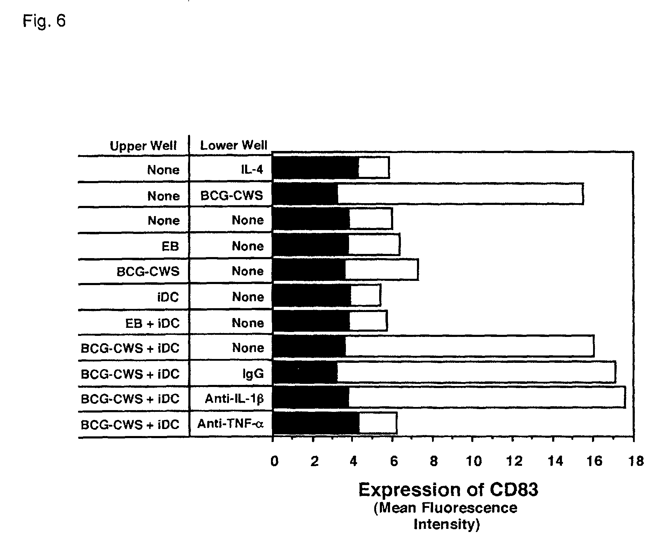

Figure 6 is a graph showing the levels of CD83 in the lower well CD in the transwell system.

A factor responsible for surface CD83 up-regulation secondary to BCG-CWS treatment was identified by transwell assay. iDC prepared as in Figure 1 were incubated for 2 days in transwell apparatus with GM-CSF (500 IU/ml) and reagents indicated in the figure. The cells in the lower well were harvested, and levels of CD83 were measured by flow cytometry. Values are expressed as mean fluorescence intensity. Emulsion buffer is denoted as EB. One of three experiments is shown.

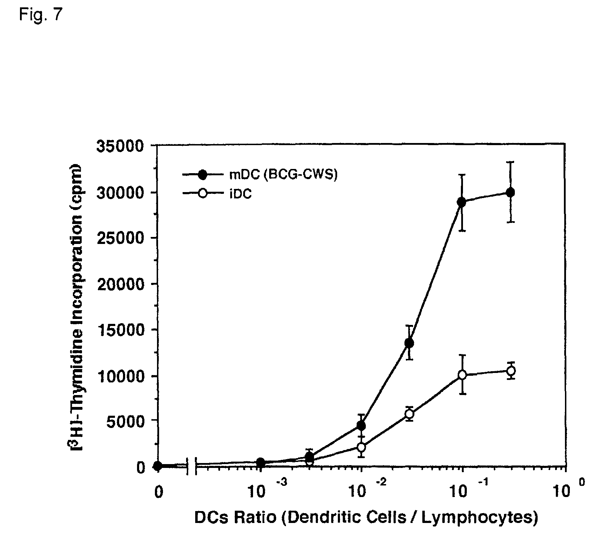

Figure 7 is a graph showing Allogenic MLR using iDC and DC^{BCG}.

Antigen presentation ability of DC was assessed by MLR. iDC as in Figure 1 were incubated for 2 days in medium, containing GM-CSF plus IL-4 or GM-CSF plus BCG-CWS. These DCs were irradiated and cultured for 4 days with allogenic lymphocytes, and [³H]-TdR incorporation was measured as described in the Methods section. Values are expressed as the means ± SD of triplicate determinations. Similar experiments were performed twice and a representative one is shown.

### BEST MODE FOR CARRYING OUT THE INVENTION

The following Examples are provided for further illustration of the present invention in detail.

### Examples

### Maturation of iDC and characterization of the matured cells

### Materials and Methods.

### 1. Reagents, ELISA kits and Antibodies

BCG-CWS material (lot No. 10-2) was prepared as described previously (17). Specifically, BCG-CWS is an insoluble residue, which is obtained by destruction step of BCG bacteria followed by purification step such as exclusion of nucleic acids and proteins, and delipidization. The following materials were obtained as indicated: FBS (fetal bovine serum) from Bio Whittaker (Walkersville, MD), human AB serum from ICN Biomedicals, Inc. (Aurora, OH), GM-CSF (granulocyte-macrophage colony stimulating factor), IL-1β, and IL-4 from Pepro Tech EC, LTD.(London, UK), TNF-α from Gibco BRL (Rockville, MD), LPS (lipopolysaccharide) (E. coli 0127:B8) from Difco Laboratories (Detroit, MI), [³H]-thymidine from NEN Life Science Products, Inc. (Boston, MA), GMDP (N-Acetyl-D-glucosaminyl-(β1-4)-acetyl-L-alanyl-D-isoglutamine) from Calzyme Laboratories, Inc. (San Luis Obispo, CA).

The following ELISA kits were obtained as indicated: GM-CSF, IFN-_{γ}, IL-1β, IL-6, and TNF-α from Amersham Pharmacia Biotech (Buckinghamshire, UK), total IL-12 (p40 plus p70) from Genzyme Co. (Cambridge, MA), IL-12 p70 from Endogen, Inc. (Woburn, MA), active IL-18 from Medical & Biological Laboratories Co., LTD (Nagoya, Japan), endotoxin specific assay kit (Endospecy ES-6 set) from Seikagaku Co. Ltd. (Tokyo, Japan). The following antibodies were obtained as indicated: anti-CD1a (B17.20.9), anti-CD80 (MAB104), and anti HLR-DR (Immu-357) from Immunotech. (Marseille, France), anti-CD11c (S-HCl-3) from Becton Dickinson monoclonal center, Inc. (Mountain View, CA), anti-CD14 (UCHM-1), IgG1 (MOPC-21), and IgG2a (UPC-10), IgG2b (MOPC-141) from Sigma Chemical Co. (Saint Louis, MO), anti-CD40 (5C3) and anti-CD64 (10.1) from PharMingen (San Diego, CA), anti-CD71 (Ber-T9) from DakoPatts (Glostrup, Denmark), anti-CD83 (HB15A) from Cosmo Bio Co. (Tokyo, Japan), anti-CD86 (BU63) from Ancell Co. (Bayport, MN), anti-TNF-α, anti-IL-1β, and anti-IL-12 (clone C8.6) from Genzyme Co. (Cambridge, MA), FITC (fluorescein isothiocyanate)-labeled goat anti-mouse IgG F(ab')₂ from American Qualex Manufactures (San Clemente, CA).

### 2. BCG-CWS preparation

Oil-in-water emulsion forms of BCG-CWS were used throughout this study (18,20). The dried BCG-CWS was resuspended at 1 mg/ml in an emulsion buffer (PBS containing 1% drakeol and 1% Tween-80) with a Potter's homogenizer, and the suspension was sterilized by heating 30 min at 60 °C. For FITC labeling of BCG-CWS, the dried BCG-CWS was resuspended in 50mM HEPES-buffered saline (HBS), pH 8.5, at a concentration of 1 mg/ml. Thereafter, 10 µl of 10 mg/ml FITC in DMSO was added to the suspension, and the mixture was incubated for 15 min at 37 °C. The FITC-labeled BCG-CWS was collected by centrifugation (15,000 rpm, 10 min), and washed once with HBS (pH 7.0). The FITC-BCG-CWS was resuspended in an emulsion buffer at 1 mg/ml with a Potter's homogenizer, and the suspension was sterilized by heating for 30 min at 60 °C.

### 3. Cells

Peripheral blood mononuclear cells (PBMC) were isolated by standard density gradient centrifugation with Ficoll-Paque (Amersham Pharmacia Biotech AB) from the heparinized whole blood or the concentrated leukocyte fraction of normal healthy donors. CD14⁺ monocytes were separated from the PBMC by anti-CD 14-coated microbeads and MACS cell separation columns (Miltenyi Biotec GmBH) in which a magnet was utilized. Immature DC (iDC) were generated from monocytes (5x10⁵ cells/ml) cultured for 6 days in RPMI-1640, containing 10% heat-inactivated fetal bovine serum, 500 IU/ml GM-CSF, and 100 IU/ml IL-4 (26), with a medium being changed every 3 days. Lymphocytes for MLR (mixed lymphocyte reaction) were prepared from fresh PBMC that were depleted of monocytes by anti-CD14-coated microbeads and MACS columns. CD4⁺ and CD8⁺ T cells were also separated by the MACS system.

### DC maturation

iDC were prepared as described above. These cells were further cultured at 5x10⁵ cells/ml for 2 days in RPMI-1640 containing 10% heat-inactivated fetal bovine serum and 500 IU/ml GM-CSF with either one of 100 lU/ml IL-4, 100 ng/ml IL-1β, 100 IU/ml TNF-α, 10 ng/ml LPS, and 15 µl/ml emulsion buffer (vehicle of BCG-CWS), or 15 µg/ml BCG-CWS. After 2 days, the adherent cells were collected by gentle pipetting in PBS containing 10 mM EDTA.

### Phagocytosis assay by FACS

iDC were cultured for six (6) days (5x10⁵ cells/ml) as described above and were incubated with 15 µg/ml FITC-BCG-CWS in RPMI-1640 containing 10% heat-inactivated fetal bovine serum and 500 IU/ml GM-CSF at 37 °C for 0.5 hours or 7 hours. The cells were harvested at 4 °C by gentle pipetting in PBS containing 0.9 mM CaCl₂, 0.5 mM MgCl₂, 0.1% sodium azide, and 0.1% BSA, and washed with the same buffer. These cells were analyzed by flow cytometry (FACSCalibur, Becton-Dickinson). Total fluorescence reflected bound and phagocytosed FITC-BCG-CWS. For quenching the fluorescence of uningested FITC-BCG-CWS, the cell suspension was mixed in an equivalent amount of 50 mM acetate buffered saline (pH 4.5) containing 2 mg/ml trypan blue, and analyzed by flow cytometry (FACS) (27). The levels of fluorescence reflected phagocytosed FITC-BCG-CWS. Fluorescence analysis was also performed with a fluorescence microscope (Olympus, IX-70, BX-60). The fluorescence of extracellular FITC-BCG-CWS was completely quenched by this analysis.

### FACS analysis of cell surface antigens

The cells were resuspended in PBS containing 0.1% sodium azide and 0.1% BSA, and then the suspension was incubated for 30 min at 4 °C together with a saturated concentration of monoclonal antibodies. The cells were washed and counterstained with FITC-labeled goat anti-mouse IgG F(ab')₂ for 30 min at 4 °C. Fluorescence intensity was then determined by FACS analysis.

### ELISA

The DC culture supernatants were collected, cleared by centrifugation, and stored at -30 °C. Concentrations of IL-1β, IL-6, IL-12 p40, IL-12 p70, active IL-18 (type 1), GM-CSF, IFN-γ, and TNF-α were measured by commercial ELISA kits as described above. The concentration of inactive IL-18 (type 2) was quantified by a quantitative ELISA system with a capture antibody and a detection antibody which had been established in our laboratory, using a coloring kit.

### Transwell assay

Conventional iDC (3.5x10⁵ cells/well) were cultured for 2 days on the upper (100 µl) or lower (600 µl) wells of a transwell apparatus (Coming Costar Co.; 6.5 mm diameter, 0.4 µm pore size, polycarbonate membrane) in RPMI-1640 containing 10% heat-inactivated FBS and 500 IU/ml GM-CSF with either one of IL-4 (100 IU/ml), emulsion buffer (15 µl/ml), BCG-CWS (15 µg/ml), IgG1 (MOPC-21) (10µg/ml), and anti-IL-1β (10 µg/ml), or anti-TNF-α (10 µg/ml). Cells on the lower well were harvested at 4 °C by gentle pipetting in PBS containing 10 mM EDTA, and were analyzed by FACS as described above.

### Mixed lymphocytes reactions (MLR) assay and autologous T cell proliferation assay

iDC for MLR were generated from monocytes (5x10⁵ cells/ml) that had been cultured for 6 days in RPMI-1640 containing 10% heat-inactivated human AB serum, 500 IU/ml GM-CSF, and 100 IU/ml IL-4, and then cultured for 2 days in the same medium (iDC) or one containing 15 µg/ml BCG-CWS instead of IL-4 (DC^{BCG}). iDC and DC^{BCG} were irradiated (3,000 rad, ¹³⁷Cs source) and cultured for 4 days with 2x10⁵ allogenic lymphocytes in 96-well cell culture plates in 200 µl RPMI-1640 containing 10% human heat-inactivated AB serum. During the last 24 hours of the culturing, the half of the medium was replaced with a fresh medium containing [³H]-thymidine (1 µCi/well). Then, the cells and medium were harvested separately with a cell harvester and the radioactivity was measured by a liquid scintillation counter (Aloca).

In other experiments, autologous CD4⁺ and CD8⁺ T cells were used instead of allogenic lymphocytes, and cell proliferation was determined by the same assay (28).

Results obtained in the above studies are provided below.

### Ability of BCG-CWS to mature iDC

Non-adherent iDC can be generated through treatment of peripheral blood monocytes with GM-CSF and IL-4 *in vitro* (23-25). Further maturation of iDC to mDC can be mediated by treatment with various regents such as IL-1β, TNF-α and LPS (29), although the maturation stages attained by these reagents are not always comparable.

The inventors added BCG-CWS to each of these maturation stages to test its ability to induce APC (antigen presenting cell). BCG-CWS was not a substitute for any factor that was essential for differentiation of monocytes to iDC. As previously reported (30), monocytes were differentiated into macrophages through the treatment with BCG-CWS. Interestingly, BCG-CWS treatment of iDC generated mDC having a unique profile. A typical morphological feature of iDC was non-adhesive dendritic structure (Fig. 1a). Further stimulation with IL-1β or an emulsion buffer only gave rise to extension of pseudopodia with adhesion (Fig. 1c, f). Aggregations of cells were observed in GM-CSF- or LPS- treated iDC without marked alteration of cell shape (Fig. 1b, e). TNP-α-treatment of iDC resulted in a mixture of cells with pseudopodia or elongated cell shape (Fig. 1d). In the case of BCG-CWS, iDC were converted to cells that showed a typical elongated shape (Fig. 1g), which resembled the shape observed in TNF-α-treated cells.

### Direct binding of BCG-CWS to DC (BCG-CWS is not bound to lymphocytes)

The binding properties of BCG-CWS to iDC were analyzed with FITC-labeled BCG-CWS. FITC-labeled BCG-CWS bound efficiently to iDC within 30 min as determined by flow cytometric analysis (Fig. 2a). The labeled particles were not phagocytosed because the fluorescence was quenched by the addition of trypan blue (Fig. 2b). Several hours later, most of the attached particles had been phagocytosed as judged by flow cytometry (Fig. 2c, d). The binding of labeled particles and the intracellular uptake of them into iDC attained during the 7 hours period was also verified by a fluorescence microscope (Fig. 1h) The same field observed under a phase-shift microscope is shown in Fig. 1i. Some FITC-labeled BCG-CWS particles bound to the surface of cells.

### Maturation of iDC by BCG-CWS

Previous studies of surface markers have suggested that during maturation from monocytes to iDC, CD1a levels are elevated, while levels of CD14 and CD64 are decreased on cells (25). Treatment of monocytes with BCG-CWS, however, did not induce up-regulation of CD1. iDC, resulting from IL-4 and GM-CSF treatment, showed high levels of CD1a, HLA-DR, CD40, CD71, CD80 and CD11c, and low levels of CD14 and CD64 as compared to monocytes (Fig. 3), while the level of CD83 was unchanged.

Interestingly, the typical surface marker profiles of the iDC were altered by treatment with BCG-CWS. Two days after the BCG-CWS treatment, CD40, CD71, CD80 and CD86 levels were further increased. Strikingly, CD83, a marker of mature DC, appeared on the cell surface by BCG-CWS treatment, though iDC express this marker only minimally (Fig. 3). Taken together, these flow cytometric data suggest that BCG-CWS may serve as an inducer of DC maturation.

### The levels of receptor/costimulatory molecules on DC treated with BCG-CWS

The levels of surface markers on DC after the BCG-CWS treatment were compared to those of typical mDC matured with IL-1β (Fig. 4). BCG-CWS induced slightly more CD86 and slightly less CD80 than the IL-1β treatment. CD40, CD71 and CD83 levels were also increased to similar extents in these two DC lineages. Treatment with another DC maturation inducer TNF-α, showed an effect similar to that of BCG-CWS on the surface levels of these molecules. Although the effects of LPS treatment were similar to that of BCG-CWS, 3-5 fold greater increases in the levels of these markers were seen after the addition of LPS compared to BCG-CWS or TNF-α. The levels of contaminating LPS in the medium containing BCG-CWS, the emulsion buffer, and saline, were 10.2 ± 0.2 pg/ml, 8.7 ± 0.2 pg/ml and 6.3 ± 0.2 pg/ml, respectively, suggesting that the effect of LPS contamination on DC maturation was negligible. Although N-Acetyl-D-glucosaminyl-(β1-4)-acetyl-L-alanyl-D-isoglutamine (GMDP) is the component responsible for the adjuvant activity in BCG-CWS (31), synthetic GMDP (15 µg/ml) showed no effect on these markers.

### Cytokine induction in iDC by BCG-CWS

The inventors next determined the levels of cytokines secreted by DC into the culture media (Fig. 5). DC secreted very low levels of IL- 1β, TNF-α and IL-12 p40 at each time point during culturing. Monocytes released a large amount of IL-6 by treatment with IL-4 plus GM-CSF, although this ability was abrogated after the maturation into DC. This cytokine liberation profile was markedly altered by BCG-CWS treatment, which induced the secretions of TNF-α, IL-6 and IL-12 p40 in the cells treated with IL-4+GM-CSF for more than 6 days, namely iDC. Although the levels of the liberated cytokines differed depending on the IL-4+GM-CSF-culturing period, the levels of IL-6 and IL-12 p40 were both higher. It is notable that the time-course curves of IL-12 p40 and TNF-α paralleled that of CD83 expression level (Fig. 3). No GM-CSF and IFN-γ were detected in the culture supernatant. The concentration of IL-12 p70 was also minimal despite of BCG-CWS treatment (Table 1).

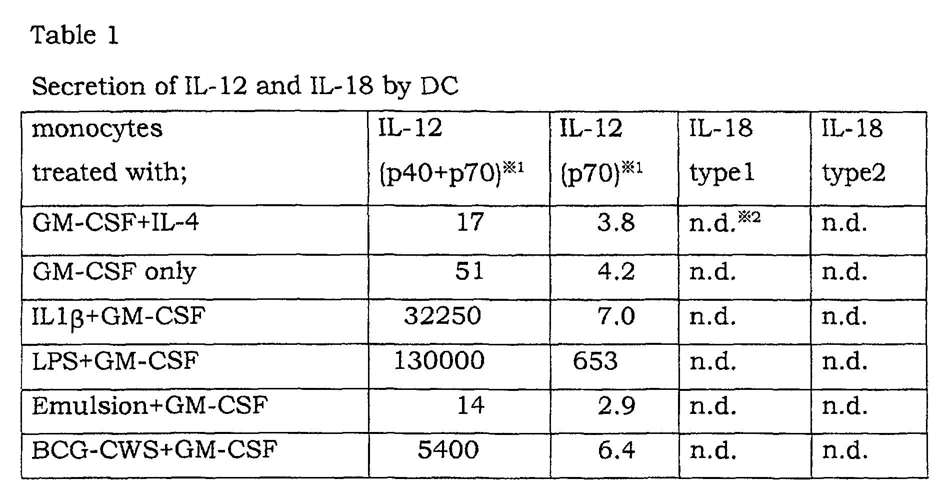

One of the three experiments is shown. 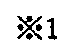 pg/ml. 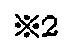 not detected. Similar results were also obtained in DC that had been treated with TNF-α or IL-1β. In contrast, DC matured with LPS produced high levels of IL-12 p70, but barely released both type 1 and type 2 of IL-18 into the medium. These cytokine profiles of DC treated with BCG-CWS, together with the up-regulation of surface CD83 and CD86 levels indicate that BCG-CWS is an inducer of DC maturation.

### Autocrine activation of DC by TNF-α induced by BCG-CWS

We next determined the factors which were either directly or indirectly responsible for DC maturation after the BCG-CWS treatment. A transwell apparatus was employed for this analysis. Stimulator or mediator sources (including iDC) were placed in the upper wells and iDC were added to the lower wells with or without antibodies against mediators. The cells in the lower wells were analyzed by flow cytometer using anti-CD83 monoclonal antibody (Fig. 6). CD83 was not induced, when either emulsion buffer or BCG-CWS which could not pass through the intercepting membrane, was added to the upper wells. iDC in the lower wells also did not express CD83, when the upper wells were filled with iDC or iDC plus an emulsion buffer. The iDC in the lower well expressed CD83 only when iDC and BCG-CWS were simultaneously added to the upper well. The expression of CD83 on the iDC in the lower well was not suppressed by the addition of anti-IL-1β to the lower well but was abrogated by the addition of anti-TNF-α to the lower well. These results suggest that BCG-CWS-mediated maturation of iDC (DC^{BCG}) is induced by TNF-α secreted by iDC per se.

If this is the case, iDC can be activated in an autocrine fashion by BCG-CWS-inducible TNF-α. In fact, direct inhibition of the conversion of iDC into mDC was observed by the addition of anti-TNF-α but not anti-IL-1β. Thus, the direct contact of BCG-CWS or an emulsion buffer to iDC is not a factor in CD83 expression. IL-12 marginally affected iDC maturation, since neither anti-IL-12 nor recombinant IL-12 p40 affected the levels of CD83 expression in DC^{BCG} or iDC, respectively.

### Antigen presenting (AP) and phagocytic ability of iDC, mDC and DC^{BCG}

Phagocytic activity was determined with FITC-labeled BCG-CWS (Table 2).

**Table 2**

| Phagocytic activity of DC | |
|---|---|
| Type of DC | Activity of BCG-CWS-Phagocytosis ) |
| | (Mean Fluorescence Intensity) |
| iDC | 25.05±4.31 (n=4) |
| TNF α-derived mDC | 6.13±2.01 (n=4) |
| DO^{BCG} | 15.90±3.83 (n=6) |

iDC exhibited high BCG-CWS-phagocytic activity while mDC prepared with TNF-α largely lost the activity. DC^{BCG}, however, still retained phagocytic activity compared to TNF-α-derived mDC.

The AP activity of iDC and DC^{BCG} was assessed by MLR. iDC facilitated an increase of allogenic lymphocytes while addition of BCG-CWS showed approximately a 3-fold more effective increase in lymphocytes. DC^{BCG} treatment amplified lymphocyte proliferation but did not increase the sensitivity to target cells (Fig. 7). In other preliminary experiments, iDC could not activate autologous CD8⁺ T cells, while DC^{BCG} activated both autologous CD4⁺ and CD8⁺ T cells in the presence of FBS.

The references that are referred hereto are provided below.

### References:

1. Rescigno, M. et al., 1999, Immunol. Today 20:200-203
2. Fearon, D. T. et al., 1996, Science 272:50-53
3. Medzhitov, R. et al., 1997, Cell 91:295-298
4. Kaufmann, S. H. E. et al., 1993, Trends Microbiol. 1:2-5
5. Inaba, K. et al., 1993, J. Exp. Med. 178:479-488
6. Thurnher, M. et al., 1997, Int. J. Cancer 70:128-134
7. Henderson, R. A. et al., 1997, J. Immunol. 159:635-643
8. DeVita, V. T. Jr. et al., 1991, Biological therapy of cancer., ed. by J. B. Lippincott, Philadelphia. pp. 1-800
9. Mathe, G. et al., 1973, Natl. Cancer Inst. Monogr. 39:107-113
10. Alexandroff, A. B. et al., 1999, Lancet 353:1689-1694
11. Ransom, J. H. et al., 1993, Int. J. Cancer 54:734-740
12. Vermorken, J. B., 1999, Lancet 353:345-350
13. Minden, P. et al., 1980, Cancer Res. 40:3214-3217
14. Hawrylko, E., 1975, J. Natl. Cancer Inst. 55:413-423
15. Lamensans, A. et al., 1975, Proc. Natl. Acad. Sci. USA 72:3656-3660
16. Bartlett, G. L. et al., 1976, J. Natl. Cancer Inst. 57:1297-1303
17. Azuma, I. et al., 1974, J. Natl. Cancer Inst. 52:95-101
18. Zbar, B. et al., 1974, J. Natl. Cancer Inst. 52:1571-1577
19. Yamamura, Y. et al., 1976, Ann. N. Y. Acad. Sci. 277:209-227
20. Hayashi, A., 1994, Proc. Japan Acad. 70(B):205-209
21. Hayashi, A., et al., 1998, Proc. Japan Acad. 74(B):50-55
22. Romani, N. et al., 1994, J. Exp. Med. 180:83-93
23. Sallusto, F., 1994, J. Exp. Med. 179:1109-1118
24. Zhou, L., 1996, Proc. Natl. Acad. Sci. USA 93:2588-92
25. Pickl, W. F. et al., 1996, J. Immunol. 157:3850-3859
26. Palucka, K. A. et al., 1998, J. Immunol. 160:4587-4595
27. Takizawa, F. et al., 1996, FEBS Lett. 397:269-72
28. Zarling, A. L. et al., 1999, J. Immunol. 162:5197-5204
28. Banchereau, J. et al., 1998, Nature 392:245-52
29. Begum, N. A. et al., 1999, Biochem. Biophys. Res. Commun. 256:325-9
30. Ellouz, F. et al., 1974, Biochem. Biophys. Res. Commun. 59:1317-25
31. Knutson, K. L. et al., 1998, J. Biol. Chem. 273:645-652
32. Kaplan, G., 1987, J. Immunol. 138:3028-3034
33. Barnes, P. F. et al., 1992, J. Immunol. 149:541-547
34. Grand-Perret, T., 1986, Eur. J. Immunol. 16:332-338
35. Oswald, I. P. et al., 1997, Infect. Immun. 65:1364-1369
36. Yamamoto, S. et al., 1988, Jpn. J. Cancer Res. 79:866-873
37. Jakob, T. et al., 1998, J. Immunol. 161:3042-3049
38. Freund, J., 1956, Adv. Tuberc. Res. 7:130-148
39. Yang, R. B. et al., 1998, Nature 395:284-288
40. Medzhitov, R. et al., 1997, Nature 388:394-397
41. Poltorak, A. et al., 1998, Science 282:2085-2088
42. Schwandner, 1999, J. Biol. Chem. 274:17406-17409
43. Adachi, O. et al., 1998, Immunity 9:143-150
44. Hoshino, K. et al., 1999, J. Immunol. 162:3749-3752
45. Yasumoto, K. et al., 1979, Cancer Res. 39:3262-3267

### INDUSTRIAL APPLICABILITY

As shown above, it is apparent that the present invention provides matured dendritic cells that maintain a phagocytic ability, and a process for maturing immature dendritic cells into matured dendritic cells that maintain a phagocytic ability, which comprises employing a Gram positive bacteria-CWS, and it further provides a process for inducing the expression of TNF-α, CD40, CD71, CD83, CD80, and/or CD86 in dendritic cells, which comprises employing a Gram positive bacteria-CWS. The present invention also provides a composition for accelerating the maturation of immature dendritic cells, or a composition for accelerating the induction of TNF-α, IL-12p40, and/or IL-6, and further a composition for accelerating the expression of CD40, CD71, CD83, CD80, and CD86, all of which comprises a Gram positive bacteria-CWS as an active ingredient. Also, the present invention provides an immune adjuvant or a composition for immunotherapy of a cancer (desirably, it is used together with an anti-cancer vaccine), which comprises a matured dendritic cell that maintains a phagocytic ability.

Cancer antigens used in an anti-cancer vaccine are exemplified by those described in *SAIBOUKOGAKU* (Cellular Engineering) 18(9),1379-1388(1999). Specific examples includes CEA peptide, MUC-1, HER2p63 that is a peptide derived from oncogene HER2 protein, or the like.

## Claims

1. A matured dendritic cell that maintains a phagocytic ability.

2. The cell of claim 1, in which the cell is matured with a Gram positive bacteria-CWS.

3. The cell of claim 2, in which the Gram positive bacterium is BCG.

4. A process for maturing an immature dendritic cell, which comprises employing a Gram positive bacteria-CWS, said process providing a matured dendritic cell that maintains a phagocytic ability.

5. The process of claim 4, in which a Gram positive bacterium is BCG.

6. A process for inducing the expression of TNF-α, CD40, CD71, CD83, CD80, and/or CD86 in dendritic cells, which comprises employing a Gram positive bacteria-CWS.

7. The process of claim 6, in which the Gram positive bacterium is BCG.

8. A composition for accelerating the maturation of immature dendritic cells, which comprises a Gram positive bacteria-CWS as an active ingredient.

9. The composition of claim 8, in which the Gram positive bacterium is BCG.

10. A composition for accelerating the induction of TNF-α, IL-12p40, and/or IL-6, which comprises a Gram positive bacteria-CWS as an active ingredient.

11. The composition of claim 10, in which the Gram positive bacterium is BCG.

12. A composition for accelerating the expression of CD40, CD71, CD83, CD80, and CD86, which comprises a Gram positive bacteria-CWS as an active ingredient.

13. The composition of claim 12, in which the Gram positive bacterium is BCG.

14. An immune adjuvant which comprises a matured dendritic cell that maintains a phagocytic ability.

15. The immune adjuvant of claim 14, in which the matured dendritic cell is obtained by employing a Gram positive bacteria-CWS.

16. The immune adjuvant of claim 15, in which the Gram positive bacterium is BCG.

17. A composition for immunotherapy of a cancer, which comprises a matured dendritic cell that maintains a phagocytic ability.

18. A composition for immunotherapy of a cancer, which comprises a matured dendritic cell that maintains a phagocytic ability, said composition being used together with an anti-cancer vaccine.

19. The composition of claim 17 or 18, in which the matured dendritic cell is obtained by employing a Gram positive bacteria-CWS.

20. The composition of claim 19, in which the Gram positive bacterium is BCG.

21. A composition for accelerating the maturation of immature dendritic cells, which comprises mycolic acid, arabinogalactan, or peptidoglycan, or a mixture thereof.
